# EUROPÄISCHE PATENTSCHRIFT

(11) **EP 4 622 580 B1**
(45) Veröffentlichungstag und Bekanntmachung des Hinweises auf die Patenterteilung: **05.08.2026**
(21) Anmeldenummer: 24726634.9
(22) Anmeldetag: 16.05.2024
(51) Int. Cl.: A61B 34/20, A61B 34/30

(54) **CHIRURGISCHES ASSISTENZSYSTEM**
SURGICAL ASSISTANCE SYSTEM
SYSTÈME D'ASSISTANCE CHIRURGICALE

(30) Priorität: 17.05.2023 DE 102023113045
(43) Veröffentlichungstag der Anmeldung: 01.10.2025
(73) Patentinhaber: B. Braun New Ventures GmbH, 79110 Freiburg im Breisgau (DE)
(72) Erfinder: BEYL, Tim, 79115 Freiburg im Breisgau (DE); SARVESTANI, Amir, 79104 Freiburg (DE); STAWIASKI, Jean, 79117 Freiburg (FR)
(74) Vertreter: Winter, Brandl - Partnerschaft mbB
(86) Internationale Anmeldenummer: PCT/EP2024/063603
(87) Internationale Veröffentlichungsnummer: WO 2024/236147

(56) Entgegenhaltungen:
- EP-A1- 3 922 203
- DE-A1- 102021 102 274
- DE-A1- 102021 128 478
- US-A1- 2022 168 048

## Beschreibung

### Technisches Gebiet

Die vorliegende Offenbarung betrifft ein chirurgisches Assistenzsystem, insbesondere ein neurochirurgisches Assistenzsystem, mit einem navigierten, chirurgischen Roboter zur Verwendung bei einem chirurgischen Eingriff an einem Patienten. Der Roboter hat hierfür eine Roboterbasis als lokalen Anbindungspunkt des Roboters und insbesondere als lokales, ortsfestes Koordinatensystem oder Referenzkoordinatensystem. An der Roboterbasis ist ein beweglicher bzw. aktiv bewegbarer Roboterarm mit zumindest einem Roboterarm-Segment angebunden. Zudem hat das Assistenzsystem ein Visualisierungssystem als dessen Endeffektor, insbesondere mit einer oder mehreren Kameras, das an dem Roboterarm, insbesondere an einer endständigen Seite des Roboterarms, angebunden, insbesondere gelagert ist. Das Visualisierungssystem ist angepasst, zumindest eine zeitaktuelle, korporale, vorzugsweise intrakorporale, Aufnahme des Patienten zu erstellen und, vorzugsweise digital, bereitzustellen. Beispielhaft beschrieben sind zudem ein computerimplementiertes Verfahren zur Steuerung sowie ein computerlesbares Speichermedium sowie Computerprogramm.

### Technischer Hintergrund

Im Bereich der Medizin bzw. Medizintechnik gewinnt eine Automatisierung mit einhergehender Einbindung digital steuerbarer technischer Vorrichtungen weiter an Bedeutung. Bei einem chirurgischen Eingriff werden vermehrt Roboter eingesetzt, welche insbesondere einen präzisen minimalinvasiven Eingriff unterstützen. Hierbei wird der Roboter nicht nur als alleinstehender Roboter vorgesehen, der einzig die Operation durchführt, sondern der Roboter wird verstärkt als kollaborativer Roboter (Cobot), also als assistierender bzw. unterstützender Roboter, direkt im Eingriffsfeld eingesetzt, welcher mit einem medizinischen Fachpersonal, insbesondere dem Chirurgen, interagiert.

Zu solch roboterunterstützen, chirurgischen Eingriffen, insbesondere neurochirurgischen Eingriffen am Gehirn, werden in der Regel verschiedene Visualisierungs- und Messsysteme zur visuellen, physiologischen und funktionellen Beurteilung des Eingriffsgebietes und als Entscheidungshilfe eingesetzt. Zu den klassischen Daten, die dazu während des Eingriffs erfasst werden, gehören die Aufnahmen eines im Eingriffsgebiet platzierten Visualisierungssystems, beispielsweise eines chirurgischen Mikroskops und/oder Endoskops.

Bei herkömmlichen Assistenzsystemen, wird das Instrument mittels am Instrument befestigter optischen Referenzmarkern getrackt, insbesondere durch einen Starrkörper mit vier Kugeln, welche im Raum nachverfolgt werden können. In der Neurochirurgie wird entweder ein Navigationspointer oder ein Saugrohr als Instrument genutzt. Allerdings beeinflussen und verschlechtern die Referenzmarker die Ergonomie aufgrund ihrer Größe und ihres Gewichtes, verursachen Zusatzaufwände und Zusatzkosten und sind aufwendig in der Anwendung. Darüber hinaus verursachen sie Sichtlinienprobleme, da das Mikroskop häufig die Sicht zwischen der Navigationskamera und dem Instrument versperrt, sowie auch der Starrkörper mit den Referenzmarkern im Sichtfeld des Mikroskops eine Sicht verdeckt. Das bedingt dann eine manuelle Korrekturbewegung der Navigationskamera, wodurch eine automatische Folgefunktion, das heißt ein automatisches Nachbewegen des Visualisierungssystems in Abhängigkeit der Instrumentenbewegung, entwertet wird. Ein zusätzliches Problem ist, dass die optische Achse des Visualisierungssystems mit der Arbeitsachse des Instrumentes koinzident (koaxial) ist. Dadurch wird das Sichtfeld des Visualisierungssystems stark vom Schaft des Instrumentes beeinflusst, und teilweise versperrt.

Die Druckschrift DE 10 2021 128 478 A1 zeigt ein chirurgisches Navigationssystem zur Navigation bei einem chirurgischen Eingriff bei einem Patienten zur Nachverfolgung/ zum Tracken von zumindest einem medizinischen Instrument.

Die Druckschrift EP 392 22 03 A1 zeigt ein Kamera-Tracking-System für die computergestützte Navigation während einer Operation. Das Kamera-Tracking-System umfasst eine Kameraleiste mit drei Tracking-Kameras. Eine mittlere der Tracking-Kameras ist zwischen den beiden äußeren Tracking-Kameras so angeordnet, dass sie einen Abstand von einer Linie aufweist, die durch die Mittelpunkte der beiden äußeren Tracking-Kameras geht.

Die Druckschrift US 2022 168 048 A1 zeigt ein System zur Verfolgung der Position eines chirurgischen Instruments, das von einem chirurgischen Robotersystem manipuliert wird, um festzustellen, ob das Instrument korrekt positioniert und ausgerichtet ist. Hierzu sind Miniatur-3D-Verfolgungskameras am Endeffektor des Robotersystems angebracht. Eine anfängliche räumliche Position und Ausrichtung des chirurgischen Instruments an einer anatomischen Oberfläche wird erfasst und ein Fortschritt des chirurgischen Instruments in den anatomischen Körperteil hinein wird mit einer der Miniaturkameras verfolgt.

### Zusammenfassung der vorliegenden Offenbarung

Es ist demgegenüber die Aufgabe der vorliegenden Offenbarung, Nachteile aus dem Stand der Technik zu vermeiden oder zumindest zu mindern und ein chirurgisches Assistenzsystem zur Verfügung stellen, welches eine besonders gute und intuitive Steuerung eines Visualisierungssystems bereitstellt. Eine Teilaufgabe kann darin gesehen werden, eine handfreie Steuerung bereitzustellen, bei der der Chirurg weiterhin beide Hände für seinen Eingriff nutzen kann, jedoch mit in seiner Hand befindlichen Instrumenten zusätzlich auch die Steuerung durchführen kann. Eine Teilaufgabe kann darin gesehen werden, mit möglichst einfachen Objekten eine Steuerung des Roboters für eine Visualisierung bei einem Eingriff durchzuführen, wobei diese Objekte insbesondere keine Sicht eines Visualisierungssystems behindern. Auch soll durch nur eine möglichst geringfügige Anpassung mit einhergehenden verbesserten Herstellungskosten sowie Vermeidung von Fehlerquellenvermeidung von bereits bestehenden Effektoren, wie medizinischer Instrumente, für einen Nutzer eine Möglichkeit einer Steuerung geschaffen werden. Eine weitere Aufgabe kann darin gesehen werden, dass mit einem Effektor eine besonders präzise Steuerung des Roboters und des Visualisierungssystems erfolgen kann. Auch soll eine Ergonomie verbessert werden.

Die Aufgaben werden hinsichtlich eines chirurgischen Assistenzsystems erfindungsgemäß durch die Merkmale des Anspruchs 1 gelöst.

Ein Grundgedanke der Offenbarung besteht also darin, ein chirurgisches Assistenzsystem zu schaffen, das ein robotisch bewegtes Visualisierungssystem aufweist, das insbesondere ein chirurgisches Mikroskop oder ein Endoskop - oder beides - umfasst, und das angepasst ist, die Bewegung des Visualisierungssystems mittels einem (medizinischen oder chirurgischen) Effektor, insbesondere einem chirurgischen Instrument, zu steuern, wobei ein (aufnahmen-)bildbasiertes Tracking genutzt wird, um eine optische Achse des Visualisierungssystem relativ zu einer Arbeitsachse des Effektors zu steuern, insbesondere nachzuführen, wobei diese Steuerung oder Nachführung kontinuierlich/permanent aktiv ist und/oder durch einen Benutzer punktuell, insbesondere bei Bedarf, aktivierbar ist.

Konkret weist ein offenbarungsgemäßes chirurgisches Assistenzsystem, insbesondere ein neurochirurgisches Assistenzsystem, einen, insbesondere navigierten, chirurgischen Roboter zur Verwendung bei einem chirurgischen Eingriff auf. Der Roboter hat eine Roboterbasis als lokalen Anbindungspunkt des Roboters und einen an die Roboterbasis angebundenen, bewegbaren Roboterarm mit zumindest einem Roboterarm-Segment, insbesondere einer Vielzahl an Roboterarm-Segmenten, welche mittels Lager, insbesondere Gelenken miteinander verbunden sind und eine aktive Konfiguration des Roboterarms ermöglichen. Am Roboterarm, insbesondere an einer endständigen Seite des Roboterarms, ist ein Visualisierungssystem (oder eine Visualisierungseinheit) als Endeffektor angebunden, insbesondere gelagert. Dieses Visualisierungssystem ist angepasst, zumindest eine zeitaktuelle, intrakorporale Aufnahme des Patienten zu erstellen und in Folge digital und somit computerlesbar bereitzustellen. Das Assistenzsystem beinhaltet zudem einen (zum Roboter separaten) chirurgischen Effektor, insbesondere ein (separates) chirurgisches Instrument, wobei der Effektor insbesondere von einem medizinischen Fachpersonal manuell führbar ist, bzw. geführt ist oder maschinell aktuiert oder gehalten ist. Ein Arbeitspunkt des Effektors ist auf oder bei einer Haupt-, Längs- oder Arbeitsachse des Effektors angeordnet. Bestimmungsgemäß ist der Arbeitspunkt in einem Sichtfeld des Visualisierungssystems angeordnet oder zumindest anordenbar, sodass das Visualisierungssystem dem Operateur anforderungsgemäß die intrakorporale Aufnahme mit dem darin abgebildeten Arbeitspunkt bereitstellt. Des Weiteren ist eine optische Referenzmarke vorgesehen, die an dem Effektor in einer mit Bezug zum Arbeitspunkt vorbestimmten Position - insbesondere mit einem vorbestimmten Abstand, insbesondere mit einer vorbestimmten Position und Orientierung - angeordnet ist. Die Referenzmarke ist dafür angepasst, dass auf Basis ihrer optischen Erfassung eine Position des Arbeitspunktes und/oder eine Orientierung der Arbeitsachse ermittelbar ist. Offenbarungsgemäß und abweichend von herkömmlichen, optischen Referenzmarken im Sinne eines "rigid-bodys", ist die Referenzmarke von einem optischen Muster gebildet, das sich entlang zumindest eines Oberflächenabschnitts des Effektors erstreckt, vorzugsweise entlang eines Oberflächenabschnitts eines Effektorgehäuses. Das Muster kann sich dabei prinzipiell an beliebiger Position am Effektor befinden, bevorzugt ist jedoch ein distaler Bereich oder Abschnitt, insbesondere in einem Umfeld des Arbeitspunktes, um eine besonders präzise Lokalisierung im Sichtfeld des Visualisierungssystems zu ermöglichen. Diese distale Anbringung des Musters erlaubt insbesondere, dass Instrumente wie Absaugvorrichtungen, Sauger oder Saugrohre vor ihrer Benutzung gebogen werden können, um sie an die Anatomie und Ergonomie anzupassen, ohne dass die Navigations-Genauigkeit beeinflusst wird. Eine weitere Komponente des Assistenzsystems ist ein Trackingsystem, insbesondere ein Navigationssystem, das offenbarungsgemäß dafür angepasst ist, die Position des Arbeitspunktes und/oder die Orientierung der Arbeitsachse in Abhängigkeit bzw. auf Basis des erfassten Musters zu ermitteln. Insbesondere besteht die Anpassung des Trackingsystems darin, dass es einen Algorithmus zum maschinellen Sehen hat, durch den auf Basis der Aufnahme und des darin enthaltenen Musters in Bezug zu einem Koordinatensystem des Visualisierungssystems die Position des Arbeitspunktes und/oder die Orientierung der Arbeitsachse ermittelbar ist. Eine Steuereinheit des Assistenzsystems ist dafür angepasst, eine Position des Visualisierungssystems und/oder eine Orientierung einer optischen Achse des Visualisierungssystems und/oder eine hyperfokale Distanz des Visualisierungssystems in Abhängigkeit der ermittelten Position des Arbeitspunktes und/oder der ermittelten Orientierung der Arbeitsachse zu steuern. Über das Trackingsystem des Assistenzsystems kann vorzugsweise zudem, insbesondere mit räumlichem Bezug zum Patienten, die Lage, also eine Position und eine Orientierung, des Visualisierungssystems, und damit die Lage der zumindest einen zeitaktuellen, korporalen Aufnahme verfolgt werden.

Die Erstreckung der als Muster ausgebildeten Referenzmarke entlang der Oberfläche oder dem Oberflächenabschnitt des ohnehin bestimmungsgemäß im Sichtfeld angeordneten oder anzuordnenden Effektors hat gegenüber einer herkömmlichen, optisch erfassbaren Referenzmarke, wie beispielsweise einem gesonderten Starrkörper/rigid body, der über den Effektor oder dessen Effektorgehäuse hinausragt, den Vorteil, dass die Referenzmarke in ihrer Sichtbarkeit nicht mehr vom Visualisierungssystem - beispielsweise dem Mikroskop - eingeschränkt werden kann, sodass kein Umstellen des Mikroskops mehr erforderlich wird, und dass das Sichtfeld des Visualisierungssystems nicht mehr von dem Starrkörper blockiert werden kann. Das Visualisierungssystem muss somit nicht mehr in seiner Position und Orientierung an eine störende Position oder Lage einer Referenzmarke angepasst werden, wie das beim Stand der Technik notwendig wird, wenn beispielsweise der rigid body ins Sichtfeld gerät.

Der Begriff "Effektor" meint in der vorliegenden Offenbarung etwa ein Gerät, ein Instrument oder ähnliches medizinisches Mittel, was bei einem Eingriff für eine Durchführung des Eingriffs eingesetzt werden kann. Insbesondere kann als Effektor angesehen werden: ein Instrument, ein medizinisches Gerät wie etwa ein Endoskop oder ein Absaugschlauch, ein optisches Gerät mit einer Visualisierungsachse, ein Pointer/ Zeiger mit distaler Spitze für eine chirurgische Navigation und weitere. Die "Arbeitsachse" des Effektors meint vorliegend insbesondere eine Längsachse des Effektors, insbesondere eines distalen Abschnitts des Effektors, etwa eine Längsachse eines Bereichs des Schneidabschnitts eines Skalpells oder eine Längsachse einer Endoskopspitze (tip) oder bei einem starren Endoskop zudem auch die Längsachse des Endoskopschafts. Auch kann die Arbeitsachse bei beispielsweise einem Dentalwerkzeug mit nicht geradlinigem distalen Wirkabschnitt und Arbeitspunkt, wie einer Sonde, im Bereich des geradlinigen Abschnitts des Instruments des Handgriffs definiert werden.

Der Begriff "Arbeitspunkt" meint vorliegend den Punkt oder Bereich des Effektors, welcher bestimmungsgemäß bei einem Eingriff eingesetzt wird. Beispielsweise ist der Arbeitspunkt bei einem Skalpell die Skalpellspitze, bei einem Pointer/Zeiger die Pointerspitze, bei einer Schere im geschlossenen Zustand deren Scheidenspitze, bei einer Pinzette deren Pinzettenspitze, eine Spitze einer Bohrmaschine oder Schneidmaschine, eine Spritzenspitze, eine Spitze eines Trokars, bei einem Endoskop die Endoskopspitze, während bei einem Dentalwerkzeug einer Sonde der Arbeitspunkt die Spitze des Instruments bildet, welche (leicht versetzt) zu der Arbeitsachse nicht den distal entferntesten Punkt bildet sondern ähnlich eines Hakens leicht in Richtung proximal "zurückverläuft".

Der Begriff "Roboterarm-Segment" bedeutet hier insbesondere ein zwischen Lagern oder Gelenken gelagertes Roboterteil des Roboterarms oder im Falle des endständigen Roboterarm-Segments insbesondere ein seriell zwischen dem Endeffektor und vorangegangenen Roboterarm-Segment (bei nur einem Roboterarm-Segment entsprechend die Roboterbasis) geschaltetes Roboterteil.

Der Begriff "Position" meint eine geometrische Position im dreidimensionalen Raum, die insbesondere mittels Koordinaten eines kartesischen Koordinatensystems angegeben wird. Insbesondere kann die Position durch die drei Koordinaten X, Y und Z angegeben werden.

Der Begriff "Orientierung" wiederum gibt eine Ausrichtung (etwa an der Position) im Raum an, insbesondere die einer Achse. Man kann auch sagen, dass durch die Orientierung eine Ausrichtung angegeben wird mit Richtungs- bzw. Drehangabe im dreidimensionalen Raum. Insbesondere kann die Orientierung mittels maximal drei Winkeln angegeben werden.

Der Begriff "Lage" umfasst sowohl eine Position als auch eine Orientierung. Insbesondere kann die Lage mittels sechs Koordinaten angegeben werden, drei Positionskoordinaten X, Y und Z sowie maximal drei Winkelkoordinaten für die Orientierung.

Vorteilhafte Ausführungsformen sind in den Unteransprüchen beansprucht und werden insbesondere nachfolgend erläutert.

Gemäß einer Weiterbildung ist die Steuereinheit dafür angepasst, in einem Nachverfolgungsmodus das Visualisierungssystem derart zu bewegen, dass die Position des Arbeitspunktes des Effektors mit einem Mittelpunkt der Aufnahme oder mit einem anderen vorbestimmten oder vordefinierten Punkt der Aufnahme zusammenfällt, sodass das Visualisierungssystem dem Arbeitspunkt des Effektors folgt, und dass die optische Achse einen vorbestimmten Winkel, insbesondere eine statische Relation, zu der Arbeitsachse aufweist und diesen bei Bewegung des Effektors beibehält. **In** diesem Nachverfolgungsmodus, welcher insbesondere nutzerseitig aktivierbar und deaktivierbar ist, kann der Effektor mit seiner Arbeitsachse ähnlich eines Joysticks als Vorgabe dienen, um einen Winkel einzustellen und ferner eine räumliche Positionierung durchzuführen. Beispielsweise kann eine Spitze eines Pointers als Arbeitspunkt angeben, welcher Punkt der Bildmittelpunkt sein soll und der Roboter verfährt das Visualisierungssystem entsprechend und folgt der Spitze des Pointers. Zudem wird über die Längsachse des Pointers als Arbeitsachse mit einem vorbestimmten Winkel, beispielsweise 45°, auch noch eine Ausrichtung der optischen Achse vorgegeben. So kann der Nutzer intuitiv und schnell einen Bereich anvisieren, insbesondere mittels eines Operationsmikroskops als Visualisierungssystem, und sich darstellen lassen, ohne die Operation unterbrechen und ohne die Hände zur Verstellung nutzen zu müssen. Durch die optische Referenzmarke muss auch das Instrument, wie etwa der Pointer, nur geringfügig geändert werden, insbesondere muss keine neue Zulassung erfolgen, da lediglich eine Oberfläche optisch markiert wird, und die angepasste Steuereinheit kann das Instrument als Eingabemittel nutzen.

Gemäß einer Weiterbildung ist die Steuereinheit dafür angepasst, ein durch eine, insbesondere initiale, optische Achse des Visualisierungssystems und durch eine, insbesondere neue, Arbeitsachse des Effektors aufgespanntes, flächiges Band, insbesondere eine aufgespannte Ebene, zu bestimmen und in diesem flächigen Band/dieser Ebene (bei Bewegung des Effektors und entsprechender Veränderung der Arbeitsachse) eine neue optische Achse des Visualisierungssystems mit einem vorbestimmten Winkel zu der neuen Arbeitsachse festzulegen und den Roboterarm für eine Anordnung des Visualisierungssystems im Raum entsprechend zu steuern.

Der das Muster aufweisende Oberflächenabschnitt des Effektors ist vorzugsweise gekrümmt ausgebildet, alternativ ist er planar. Das Muster ist vorzugsweise ein 3D-Objekt, alternativ ist es ein 2D-Objekt. Das Muster ist vorzugsweise von einem Aufdruck oder von einer Eloxierung des Oberflächenabschnitts oder durch eine Laser-Applikation gebildet. Mischformen davon sind natürlich möglich.

Vorzugsweise weist der Oberflächenabschnitt eine auf einer Regelgeometrie basierende Form, vorzugsweise eine Zylinderform, auf. Vorzugsweise ist ihr Querschnitt kreisförmig oder vieleckig, insbesondere viereckig oder fünfeckig oder sechseckig. **In** bevorzugter Weiterbildung ist das Muster parallel zum Oberflächenabschnitt ausgebildet. Alternativ hat das Muster mit Bezug zur Arbeitsachse einen vom Querschnitt des Oberflächenabschnitts abweichenden Querschnitt, beispielsweise einen polygonförmigen Querschnitt, während der Oberflächenabschnitt einen kreisförmigen Querschnitt hat. Dann bildet das Muster abgewinkelte Abschnitte aus, die in der Aufnahme mittels Bilderkennung gut ausgewertet werden können. Das Muster ist zumindest abschnittsweise punktförmig und/oder linienförmig und/oder kurvenförmig ausgebildet.

Gemäß einer Weiterbildung ist der Effektor mit einer festen, das heißt nichtwechselbaren Funktionseinheit ausgebildet. Als Funktionseinheit ist offenbarungsgemäß ein Werkzeug gemeint, das während des Eingriffs die benötigte Funktion erfüllt. Es ist beispielsweise eine Schneide, ein Bohrer oder ein bildgebendes System, beispielsweise eine Ultraschallsonde, oder dergleichen. An der Funktionseinheit ist distal (an der Spitze), oder zumindest in einem distalen Bereich, der Arbeitspunkt angeordnet.

Alternativ hat der Effektor eine Wechselaufnahme, in die eine oder eines von mehreren möglichen Funktionseinheiten oder Werkzeugen wahlweise einsetzbar ist. Das Muster ist vorzugsweise in einem Bereich der Wechselaufnahme angeordnet. Vorzugsweise weisen alle Funktionseinheiten, wenn sie in die Wechselaufnahme eingesetzt sind, den gleichen Abstand und vorzugsweise die gleiche Orientierung ihres Arbeitspunktes zu dem an der Wechselaufnahme angeordneten Muster auf. Bei einem Wechsel der Funktionseinheit entfällt dann die Notwendigkeit, in das Assistenzsystem eine Information zu einem ggf. abweichenden Abstand oder einer abweichenden Orientierung des Arbeitspunktes eingeben zu müssen.

Um jederzeit die Erfassung des Musters und die darauf aufbauende Ermittlung der Position des Arbeitspunktes und/oder der Orientierung der Arbeitsachse zu gewährleisten, ist gemäß einer bevorzugten Weiterbildung das Muster am Oberflächenabschnitt derart ausgestaltet und/oder bemessen und/oder angeordnet, dass es bei bestimmungsgemäß im Sichtfeld angeordnetem Arbeitspunkt zumindest abschnittsweise im Sichtfeld angeordnet ist.

Gemäß einer Weiterbildung ist das Muster derart ausgestaltet, dass es selbst dann noch bestimmungsgemäß - das heißt zur Ermittlung der Position des Arbeitspunktes und/oder der Orientierung der Arbeitsachse - erfassbar ist, wenn es abschnittsweise - beispielsweise durch die Hand des Operateurs - verdeckt ist.

Hierzu weist das Muster gemäß einer Weiterbildung zumindest in Richtung der Arbeitsachse eine Variation auf.

Gemäß einer Weiterbildung ist im Trackingsystem ein Modell des Musters gespeichert oder es wird von der Steuereinheit dem Trackingsystem bereitgestellt. Vorzugsweise ist das Trackingsystem dafür angepasst, die Position des Arbeitspunktes und/oder die Orientierung der Arbeitsachse des Effektors in Abhängigkeit des erfassten Musters und des gespeicherten Modells zu ermitteln.

Insbesondere wird die Aufnahme, insbesondere das Mikroskop-Bild, benutzt, um mittels Machine Vision, das heißt, maschinellem Sehen, die Position und/oder Orientierung des Instruments im Mikroskop-Koordinatensystem zu erfassen. Vorzugsweise werden das Mikroskop und der Patient durch eine externe Kamera, insbesondere der eines Navigationssystems, erfasst, wodurch eine räumliche Nachverfolgungs-Kette geschlossen ist und sich über die Steuereinheit die Position und/oder Orientierung des Instruments in einem Patienten-Koordinatensystem berechnen lässt.

Ist die Erfassung einer Rotation des Effektors um seine Arbeitsachse gewünscht oder relevant für die Steuerung des Visualisierungssystems, so weist das Muster gemäß einer Weiterbildung auch in Umfangsrichtung zur Arbeitsachse eine Variation auf.

Die Variation kann insbesondere geometrischer und/oder farblicher Art sein.

Um eine möglichst gute Erfassbarkeit des Musters unabhängig von einer Rotation des Effektors zu erreichen, erstreckt sich das Muster gemäß einer Weiterbildung vollumfänglich oder zumindest umfänglich um die Arbeitsachse.

Für den Fall, dass die Rotation des Effektors hingegen nicht relevant ist, das heißt, wenn beispielsweise das Visualisierungssystem einer Rotation des Effektors nicht folgen soll, erweist sich eine Weiterbildung als vorteilhaft, in der das Muster zur Arbeitsachse zumindest abschnittsweise rotationssymmetrisch ausgebildet ist. Ist die Rotation des Effektors hingegen relevant, insbesondere wenn das Visualisierungssystem der Rotation des Effektors nachgeführt werden soll, ist das Muster zur Arbeitsachse zumindest abschnittsweise rotationsunsymmetrisch ausgebildet.

In einer bevorzugten Weiterbildung hat das Muster Elemente, die in Richtung der Arbeitsachse beabstandet sind.

Insbesondere kann der Effektor als Muster zueinander beabstandete konzentrische Ringe um seine Arbeitsachse aufweisen, welche eine unterschiedliche Breite in Richtung der Arbeitsachse aufweisen.

Damit selbst bei abschnittsweiser Verdeckung des Musters die Position des Arbeitspunktes und die Orientierung der Arbeitsachse einfach ermitteln zu können, weist gemäß einer Weiterbildung ein Abstand der Elemente untereinander und/oder eine jeweilige Erstreckung der Elemente in Richtung der Arbeitsachse eine Variation auf.

Mit gleichem Effekt sind gemäß einer Weiterbildung wenigstens einige der Elemente zu Gruppen zusammengefasst, innerhalb derer die Elemente in Bezug auf ihren Abstand untereinander und/oder ihrer jeweiligen Erstreckung in Richtung der Arbeitsachse gleich sind, während über die Gruppen hinweg betrachtet der Abstand der Elemente untereinander und/oder die Erstreckung der Elemente eine Variation aufweist oder aufweisen.

Gemäß einer besonders bevorzugten Weiterbildung ist das Visualisierungssystem dafür angepasst ist, die Aufnahme, die das Muster zumindest abschnittsweise enthält, dem Trackingsystem bereitzustellen, wobei das Trackingsystem dafür angepasst ist, die Position des Arbeitspunktes und/oder die Orientierung der Arbeitsachse des Effektors in Abhängigkeit der Aufnahme zu ermitteln. Der Vorteil ist, dass so eine Doppelfunktion des Visualisierungssystems gegeben ist. Einerseits stellt es die von dem Operateur zur Visualisierung des OP-Gebietes benötigte Aufnahme bereit. Andererseits liefert es mit der Aufnahme den notwendigen Input für die Steuerung seiner vom Effektor abhängigen Position und/oder Orientierung und/oder fokalen Distanz.

Zur Ermittlung weist das Trackingsystem vorzugsweise einen Bilderkennungsalgorithmus auf, der angepasst ist, die Position des Arbeitspunktes und/oder die Orientierung des Effektors in Abhängigkeit der Aufnahme des Visualisierungssystems zu ermitteln. Alternativ oder ergänzend hat das Trackingsystem einen maschinellen Lern-Algorithmus zur Bilderkennung, der angepasst ist, die Position des Arbeitspunktes und/oder die Orientierung des Effektors in Abhängigkeit der Aufnahme des Visualisierungssystems zu ermitteln.

Zur Ermittlung der Position des Arbeitspunktes und/oder Lage des Effektors müssen somit keine gesonderten Tracking- oder Navigationskameras mehr vorgesehen sein. Das ist vorrichtungstechnisch einfacher, verursacht weniger Kosten und es wird weniger Bauraum benötigt, sodass im OP ein Platzangebot vergrößert wird. Natürlich kann aber ergänzend wenigstens eine gesonderte 2D- oder 3D-Kamera zur optischen Erfassung des Musters vorgesehen sein, um so die Prozesssicherheit oder Genauigkeit zur Ermittlung der Position des Arbeitspunktes und/oder der Orientierung der Arbeitsachse weiter zu steigern. Die wenigstens eine gesonderte 2D- oder 3D-Kamera kann ergänzend dazu genutzt werden, den Roboterarm mit Bezug zum Patienten zu navigieren.

Mit gleichem Grund, der Verbesserung der Prozesssicherheit oder Genauigkeit, können gemäß einer Ausführungsform ergänzende, auf alternativen Referenzmarken basierende Erfassungstechnologien, beispielsweise elektromagnetische, Röntgen-, Radar-, Laser- und/oder Lidar-basierende Technologien vorgesehen sein.

Gemäß einer Weiterbildung ist die Steuereinheit dafür angepasst, die Visualisierungseinrichtung bezüglich ihrer Position und/oder Orientierung ihrer optischen Achse und/oder ihrer hyperfokalen Distanz fortwährend, beziehungsweise kontinuierlich zu steuern. Alternativ kann die Steuereinheit dafür angepasst sein, nur auf Anforderung, beziehungsweise nur nach einer Eingabe eine entsprechende Steuerung durchzuführen. Eine dritte Variante ist, dass die Steuerung wahlweise erfolgt, das heißt wahlweise entweder fortwährend oder auf Anforderung, also in zwei frei wählbaren Modi. So ist eine kontinuierliche, sowie eine eingabebasierte Nachverfolgung bereitstellt, zwischen denen ein Nutzer umschalten kann.

Gemäß einer Weiterbildung kann eine manuell betätigbare oder eine automatisierte Anforderungseinrichtung vorgesehen sein, die dafür angepasst ist, die Anforderung auszulösen. Insbesondere sind als Anforderungseinrichtung zu nennen: ein Fußpedal und/oder ein Druckknopf, der beispielsweise am Effektor vorgesehen ist, und/oder eine Stimmerkennungseinrichtung und/oder eine Gestenerkennungseinrichtung.

Die Steuerung bezüglich der Position und/oder Orientierung erfolgt vorzugsweise durch Ansteuern des Roboters und seines wenigstens einen Roboterarms derart, dass dieser die notwendige(n) Translation(en) und/oder Rotation(en) durchführt, um das Visualisierungssystem auf die vom Effektor abhängige Position und/oder Orientierung zu bewegen.

Vorzugsweise ist die Steuereinheit dafür angepasst, die Position des Visualisierungssystems und/oder die Orientierung der optischen Achse des Visualisierungssystems und/oder die hyperfokale Distanz des Visualisierungssystems in Abhängigkeit der ermittelten Position des Arbeitspunktes und/oder der ermittelten Orientierung des Arbeitsachse derart zu steuern, dass der Arbeitspunkt in einem Zentrum oder einem Mittelpunkt oder in einem vordefinierten Punkt des Sichtfeldes angeordnet ist, und/oder die optische Achse auf einen vorbestimmten Winkel zur Arbeitsachse eingestellt ist, und/oder der Arbeitspunkt mit der hyperfokalen Distanz zusammenfällt.

Offenbarungsgemäß ist die Steuereinheit dafür angepasst, die Position des Visualisierungssystems und/oder die Orientierung der optischen Achse des Visualisierungssystems und/oder die hyperfokale Distanz des Visualisierungssystems in Abhängigkeit der ermittelten Position des Arbeitspunktes und/oder der ermittelten Orientierung des Arbeitsachse derart zu steuern, dass bei fester, also unveränderlicher Position des Visualisierungssystems die hyperfokale Distanz auf den Arbeitspunkt eingestellt wird, oder dass bei fester hyperfokaler Distanz die Position des Visualisierungssystems so eingestellt wird, sodass die hyperfokale Distanz mit dem Arbeitspunkt zusammenfällt.

In bevorzugter Weiterbildung ist die Steuereinheit auf beide der genannten Arten angepasst.

Ein im Folgenden beispielhaft beschriebenes, nicht-erfindungsgemäßes Verfahren ist zum Steuern einer Position und/oder einer Orientierung einer optischen Achse und/oder einer hyperfokalen Distanz eines Visualisierungssystems, insbesondere eines chirurgischen Assistenzsystems, das gemäß einem der vorstehend beschriebenen Aspekte ausgebildet ist, vorgesehen. Das Steuern erfolgt dabei in Abhängigkeit eines getrackten, chirurgischen Effektors. Das Verfahren hat folgende Schritte:
"Erstellen und zeitaktuelles Bereitstellen einer zeitaktuellen Aufnahme eines Eingriffsgebietes, wobei ein Arbeitspunkt des chirurgischen Effektors bestimmungsgemäß in einem Sichtfeld des Visualisierungssystems angeordnet oder anordenbar ist". Dieser Schritt erfolgt mittels des Visualisierungssystems, das an einem Roboterarm angebunden ist;
"Optisches Erfassen einer optischen Referenzmarke, oder zumindest eines Abschnittes davon, wobei die optische Referenzmarke von einem optischen Muster gebildet ist, das sich entlang zumindest eines Oberflächenabschnitts des Effektors erstreckt, und das an dem Effektor in einer mit Bezug zum Arbeitspunkt vorbestimmten Position - insbesondere einem vorbestimmten Abstand - und einer vorbestimmten Orientierung angeordnet ist, und das dafür angepasst ist, dass aus seiner optischen Erfassung eine Position des Arbeitspunktes und/oder eine Orientierung der Arbeitsachse ermittelbar ist".
"Ermitteln der Position des Arbeitspunktes und/oder der Orientierung der Arbeitsachse in Abhängigkeit des optisch erfassten Musters, insbesondere mittels maschinellem Sehen auf Basis der Aufnahme in Bezug zu einem Koordinatensystem des Visualisierungssystems". Dieser Schritt erfolgt mittels einem Trackingsystem; und
"Steuern des Visualisierungssystems bezüglich seiner Position und/oder seiner Orientierung seiner optischen Achse und/oder seiner hyperfokalen Distanz in Abhängigkeit der ermittelten Position des Arbeitspunktes und/oder der ermittelten Orientierung der Arbeitsachse". Dieser Schritt erfolgt mittels einer dafür angepassten Steuereinheit.

Vorzugsweise hat das Verfahren zudem einen Schritt "Tracking, insbesondere Navigation, des Visualisierungssystems, vorzugsweise eines chirurgischen Mikroskops und/oder eines chirurgischen Endoskops". Dieser Schritt erfolgt durch das Trackingsystem oder alternativ durch ein Navigationssystem.

Gemäß einer Weiterbildung des Verfahrens wird der Schritt "Steuern des Visualisierungssystems bezüglich seiner Position und/oder seiner Orientierung seiner optischen Achse und/oder seiner hyperfokalen Distanz in Abhängigkeit der ermittelten Position des Arbeitspunktes und/oder der ermittelten Orientierung der Arbeitsachse" fortwährend ausgeführt. Fortwährend heißt, dass das Visualisierungssystem kontinuierlich jeder Bewegung des Effektors folgt. Hierbei ist eine Weiterbildung möglich, bei der eine Dämpfung oder Glättung einer Bewegung des Visualisierungssystems erfolgt, sodass auch bei ggf. ruckartig bewegtem Effektor, dennoch ein ruhiges Bewegen des Visualisierungssystems erfolgt. Die gleiche Dämpfung oder Glättung ist natürlich für die Verstellung der fokalen Distanz möglich.

Alternativ erfolgt die Steuerung nur auf Anforderung, vorzugsweise in Abhängigkeit eines Anforderungssignals, sodass das Visualisierungssystem bezüglich Position und/oder Orientierung und/oder fokaler Distanz über einen Zeitraum statisch ist, bis das Anforderungssignal erfolgt und das Visualisierungssystem erneut am Effektor orientiert positioniert und/oder ausgerichtet und/oder in seiner fokalen Distanz verstellt wird.

Das Anforderungssignal kann manuell ausgelöst werden, beispielsweise vom Operateur. Alternativ kann das Anforderungssignal automatisiert ausgelöst werden, insbesondere in Abhängigkeit von wenigstens einer chirurgischen Kontextinformation.

Des Weiteren ist eine Weiterbildung möglich, bei der die Bewegung des Roboters, und in Folge die Bewegung des Visualisierungssystems, auf einzelne translatorische oder rotatorische der möglichen sechs Freiheitsgrade X, Y, Z, RX, RY, RZ, beschränkt wird, oder auf eine Auswahl davon, beispielsweise nur auf Rotationen oder nur auf Translationen.

Gemäß einer Weiterbildung erfolgt der Schritt "Optisches Erfassen des Musters oder des Abschnitts des Musters", indem das Visualisierungssystem die Aufnahme derart erstellt, dass darin das Muster oder zumindest der Abschnitt des Musters abgebildet wird.

Gemäß einer Weiterbildung erfolgt der Schritt "Ermitteln der Position des Arbeitspunktes und/oder der Orientierung der Arbeitsachse" mittels eines im Trackingsystem, insbesondere Navigationssystem, zur Ausführung gespeicherten Bildverarbeitungsalgorithmus, der die Aufnahme verarbeitet.

Gemäß einer Weiterbildung erfolgt der Schritt "Steuern des Visualisierungssystems bezüglich seiner Position und/oder seiner Orientierung seiner optischen Achse und/oder seiner hyperfokalen Distanz in Abhängigkeit der ermittelten Position des Arbeitspunktes und/oder der ermittelten Orientierung der Arbeitsachse" in Abhängigkeit einer Abweichung der aktuellen Position und/oder aktuellen Orientierung der optischen Achse und/oder der aktuellen hyperfokalen Distanz des Visualisierungssystems von einem Soll.

Das Soll beinhaltet insbesondere, dass der Arbeitspunkt in einem vorbestimmten Bereich des Sichtfeldes, vorzugsweise einem Zentrum oder Mittelpunkt oder einem vordefinierten Punkt, sichtbar ist, und/oder dass er in der Aufnahme abgebildet ist, und/oder dass der Arbeitspunkt mit der hyperfokalen Distanz zusammenfällt, und/oder dass die optische Achse einen vorbestimmten Winkel zur Arbeitsachse aufweist. Ist der vorbestimmte Winkel ungleich null, wird verhindert, dass der Effektor, insbesondere dessen Schaft, das Sichtfeld des Mikroskops verblockt oder teilerblockt. Vorzugsweise ist die Steuereinheit derart angepasst, dass der vorbestimmte Winkel, vorzugsweise in Abhängigkeit der Präferenzen des Bedieners, voreingestellt, angepasst und/oder verändert werden kann. Der vorbestimmte Winkel kann alternativ 0° betragen.

Gemäß einer Weiterbildung beinhaltet der Schritt "Steuern des Visualisierungssystems bezüglich seiner Position und/oder seiner Orientierung seiner optischen Achse und/oder seiner hyperfokalen Distanz in Abhängigkeit der ermittelten Position des Arbeitspunktes und/oder der ermittelten Orientierung der Arbeitsachse" einen Schritt "Ansteuern des Roboters zumindest derart, dass ein vorbestimmter Bereich des Sichtfeldes oder der Aufnahme, insbesondere ein Zentrum oder ein Mittelpunkt oder ein vordefinierter Punkt, in Deckung mit dem Arbeitspunkt bewegt wird, und dass die optische Achse auf einen vorbestimmten Winkel zur Arbeitsachse angestellt wird". Bezüglich der Anforderung, dass der Arbeitspunkt mit der fokalen Distanz zusammenfällt, ergeben sich zwei mögliche Schritte. In einer Variante wird die Distanz des Visualisierungssystems zum Arbeitspunkt festgehalten und es erfolgt ein "Einstellen der hyperfokalen Distanz derart, dass der Arbeitspunkt fokussiert wird oder mit der hyperfokalen Distanz zusammenfällt". In anderer Variante wird die hyperfokale Distanz festgehalten und es erfolgt ein "Einstellen einer Distanz des Visualisierungssystems zum Arbeitspunkt derart, dass der Arbeitspunkt fokussiert wird oder mit der hyperfokalen Distanz zusammenfällt".

Der vorbestimmte Winkel als alleiniges Soll kann aber eine eindeutige Lage der optischen Achse im Raum nicht festlegen, da die optische Achse noch um die Arbeitsachse oder eine dazu parallele Achse rotieren kann. Um die optischen Achse im Raum eindeutig im Raum zu definieren, muss diejenige Ebene definiert werden, in der die optische Achse tatsächlich liegt. Hierzu gibt es im Verfahren offenbarungsgemäß zwei Möglichkeiten:
In einer Variante wird eine Ebene erzeugt, die von der ermittelten Arbeitsachse und einem Fokuspunkt der aktuellen optischen Achse aufgespannt wird. In dieser Ebene liegt dann das Soll der neuen optischen Achse mit dem vorbestimmten Winkel zur ermittelten Arbeitsachse. In dieser Variante erfährt die optische Achse keine Rotation, sollte der Effektor um seine Arbeitsachse rotiert werden.

In anderer Variante ist das Muster derart ausgestaltet, dass aus seiner Erfassung alle Translationen und Rotationen des Effektors ermittelbar sind, also auch die Rotation um die eigene Arbeitsachse. In diesem Falle wird die optische Achse bewegt, wenn der Effektor um seine eigene Achse rotiert wird.

Für eine Mehrzahl möglicher Effektoren oder Instrumente ist davon auszugehen, dass die Eigenrotation des Effektors für die Nachführung des Visualisierungssystems nicht relevant ist. Die erste Variante ist somit die bevorzugte.

Beispielhaft ist ein computerlesbares Speichermedium oder ein Computerprogramm genannt, welches Befehle umfasst, die bei einer Ausführung durch den Computer diesen veranlassen, die Schritte des zuvor beschriebenen Verfahrens auszuführen.

Jegliche Offenbarung im Zusammenhang mit dem chirurgischen Assistenzsystem gemäß der vorliegenden Offenbarung gilt für das nicht erfindungsgemäße, beispielhaft beschriebene Verfahren, wie auch umgekehrt.

### Kurzbeschreibung der Figuren

Die Erfindung wird nachfolgend anhand bevorzugter Ausführungsformen mit Hilfe von Figuren näher erläutert. Es zeigen:
Fig. 1 eine schematische Seitenansicht eines offenbarungsgemäßen chirurgischen Assistenzsystems gemäß einer bevorzugten Ausführungsform;
Fig. 2 eine perspektivische Ansicht eines Visualisierungssystems und eines Effektors des Assistenzsystems gemäß Figur 1 ;
Fig. 3 eine Detailansicht des Effektors mit einer optischen Referenzmarke in Form eines Musters gemäß Figur 2;
Fig. 4 eine schematische Darstellung einer Abfolge von Verfahrensschritten eines nicht erfindungsgemäßen, beispielhaften Verfahrens mit dem offenbarungsgemäßen chirurgischen Assistenzsystem gemäß Fign. 1 bis 3; und
Fig. 5 eine detaillierte Ansicht aus Figur 4.

Die Figuren sind schematischer Natur und sollen dem Verständnis der Erfindung dienen. Gleiche Elemente sind mit denselben Bezugszeichen versehen. Merkmale verschiedener Ausführungsformen können untereinander ausgetauscht werden.

### Detaillierte Beschreibung bevorzugter Ausführungsformen

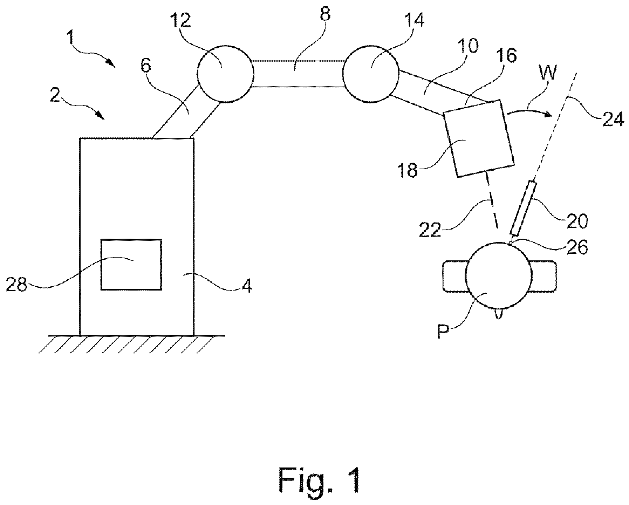

Figur 1 zeigt ein chirurgisches Assistenzsystem 1 gemäß einer bevorzugten Ausführungsform in schematischer Seitenansicht. Das Assistenzsystem 1 hat einen chirurgischen Roboter 2 mit einer Roboterbasis 4, die in der gezeigten Ausführungsform lokal fest ist. Alternativ kann sie mobil ausgeführt sein, um den Roboter 2 beispielsweise bedarfsgerecht an verschiedenen Stellen eines Operationssaals im Krankenhaus einsetzen zu können. Die Roboterbasis 4 bildet in jedem Fall einen lokalen Bezugspunkt, an dem ein mehrsegmentierter Roboterarm mit mehreren Roboterarm-Segmenten 6, 8, 10 angebunden ist, welche über Gelenke 12, 14 jeweils miteinander verbunden sind. Auf diese Weise können die Roboterarm-Segmente 8 und 10 zueinander aktiv bewegt werden und der Roboterarm 6, 8, 10, 12, 14 lässt sich im Gesamten steuern.

An einer endständigen Seite 16 des Roboterarms ist ein in der gezeigten Ausführungsform als chirurgisches Operationsmikroskop ausgeführtes Visualisierungssystem 18 des Assistenzsystems 1 befestigt. In der gezeigten Ausführungsform hat das Assistenzsystem 1 zudem einen manuell geführten, von einem Trackingsystem des Assistenzsystems 1 nachverfolgten Effektor 20, beziehungsweise ein Instrument 20. Eine optische Achse 22 des Visualisierungssystems 18 ist mit einem Winkel W gegen eine Arbeitsachse 24 des Effektors 20 angestellt. Am distalen Ende der Arbeitsachse 24 befindet sich ein Arbeitspunkt 26 des Effektors 20.

Eine Position des Visualisierungssystems 18 und eine Orientierung von dessen optischer Achse 22 kann mittels einer in der Basis 4 platzierten, speziell angepassten, zentralen Steuereinheit 28 gesteuert und eingestellt werden, wobei die spezielle Anpassung insbesondere darin besteht, dass sie in Abhängigkeit einer jeweils mittels maschinellem Sehen ermittelten Position des Arbeitspunktes 26 und/oder Orientierung der Arbeitsachse 24 erfolgt, was nachstehend tiefergehend erläutert wird.

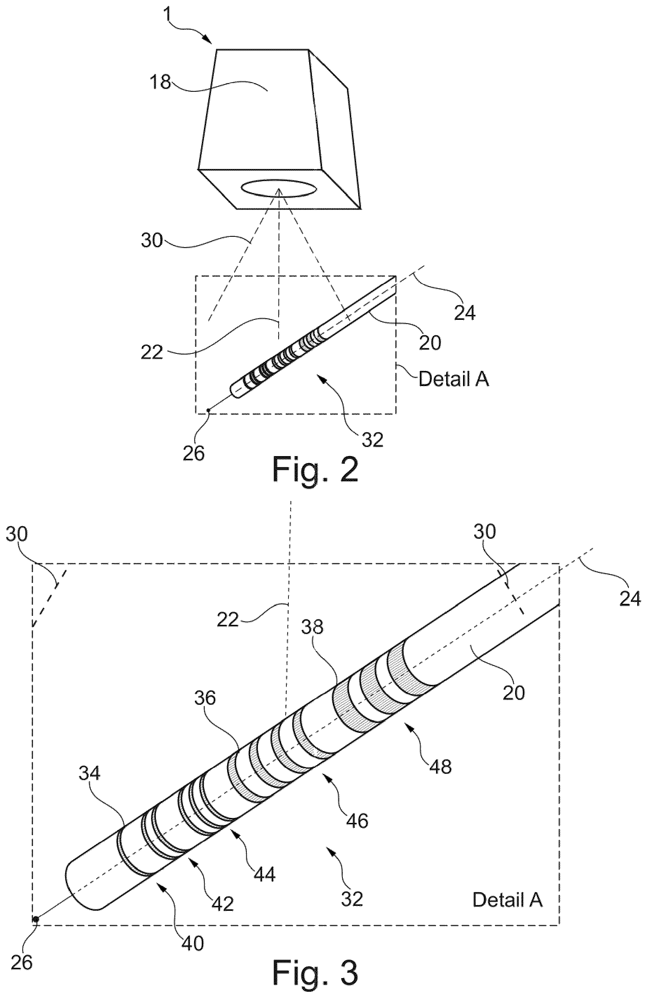

Figur 2 zeigt das Visualisierungssystem 18 mit seiner optischen Achse 22 und das Instrument 20 mit seiner Arbeitsachse 24 gemäß Figur 1 in einer perspektivischen Ansicht. Das Visualisierungssystem 18 hat ein sich auffächerndes Sichtfeld 30, in dem das Instrument 20 zumindest abschnittsweise sichtbar und dessen Arbeitspunkt 26 sichtbar platziert sind. Während des Eingriffs wird vom Visualisierungssystem 18, beispielsweise dem chirurgischen Mikroskop, fortwährend einer zeitaktuelle, intrakorporale Aufnahme vom Eingriffsgebiet des Patienten P erstellt und digital bereitgestellt.

Das Instrument 20 hat einen Schaft, dessen Oberfläche von einer Regelgeometrie und im konkreten Ausführungsbeispiel von einem Zylinder mit kreisförmigem gebildet ist. An einem zum Arbeitspunkt 26 benachbarten, distalen Endabschnitt des Schaftes, weißt ein dortiger Oberflächenabschnitt eine als optisches Muster ausgebildete Referenzmarke 32 auf. Die Referenzmarke 32 ist dafür angepasst, dass ihre optische, bildgebende Erfassung unter Anwendung eines bildverarbeitenden Algorithmus maschinellen Sehens ausgewertet werden kann, sodass hieraus die Position des Arbeitspunktes 26 und die Orientierung der Arbeitsachse 24 ermittelt werden können.

Offenbarungsgemäß erfolgt die optische Erfassung mittels dem Visualisierungssystem 18 durch dessen Aufnahme, anstatt über ein gesondertes 3D-oder 2-D-Navigationskamerasystem, wie das bei herkömmlichen Lösungen des Standes der Technik üblich ist.

Figur 3 zeigt ein gemäß Figur 2 definiertes Detail A, das einen das Muster 32 aufweisenden, distalen Oberflächenabschnitt des Instruments 20, im Bereich des Arbeitspunktes 26 vergrößert darstellt. Das Muster 32 ist in einzelne, entlang der Arbeitsachse 24 beabstandete Elemente 34, 36 und 38 unterteilt, von denen zur Wahrung der Übersichtlichkeit in Figur 3 nur einige repräsentativ mit dem entsprechenden Bezugszeichen versehen sind. Jedes einzelne der Elemente 34, 36 und 38 hat dabei einen vorbestimmten, insbesondere in der Steuereinheit 28 gespeicherten, Abstand zum Arbeitspunkt 26. Gemäß Figur 3 weist das Muster 32 in Richtung der Arbeitsachse eine Variation auf. Im konkreten Ausführungsbeispiel besteht die Variation darin, dass in genannter Richtung eine jeweilige Breite oder Erstreckung der Elemente 24, 36 und 38, und teilweise auch ihr Abstand untereinander, unterschiedlich sind. So sind die Elemente 38 am breitesten und die Elemente 32 am dünnsten, die Elemente 36 liegen in ihrer Breite im Mittelfeld. Alle Elemente 34, 36 und 38 sind zu Gruppen 40, 42, 44, 46, 48 zusammengefasst, wobei die Gruppe 40 nur ein Element 34 enthält, die Gruppe 42 zwei Elemente 34, die Gruppe 44 drei Elemente 34, die Gruppe 46 vier Elemente 36 und die Gruppe 48 drei Elemente 38. Alle Elemente 34, 36 und 38 erstrecken sich vollumfänglich und rotationssymmetrisch am Oberflächenabschnitt des Instruments 32. Aufgrund dessen, dass sich das optisch erfassbare Muster 32 in genannter Weise entlang der Oberfläche des Instruments 20 erstreckt, stellt es eine als 3D-Objekt ausgebildete Referenzmarke dar, die analog zu einem herkömmlichen rigid body, optisch erfassbar ist. Demgegenüber ist ihr Vorteil allerdings, dass sie keinen zusätzlichen Bauraum benötigt und kein zusätzliches Gewicht aufweist. Zudem kann sie in dem Sinne keine Sichtlinie versperren, da sie an der Oberfläche des ohnehin im Sichtfeld angeordneten oder anzuordnenden Instrumentes 20 vorgesehen ist.

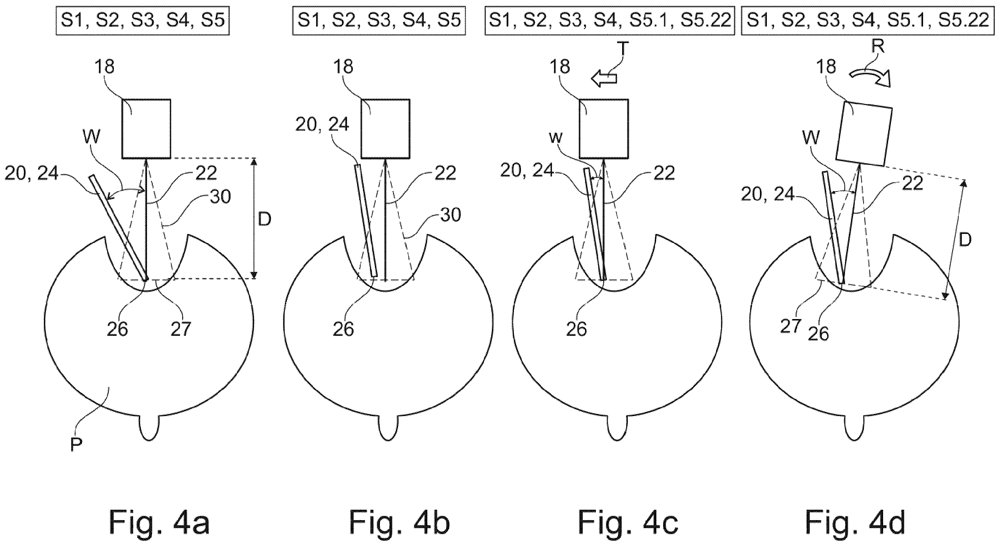

Figuren 4a bis 4d zeigen Schritte eines nicht erfindungsgemäßen, beispielhaften Verfahrens mit dem chirurgischen Assistenzsystem gemäß Figur 1, 2 und 3.

Figur 4a zeigt einen bestimmungsgemäßen Anfangszustand oder Grundzustand, in dem das Visualisierungssystem 18 bezüglich seiner Position im Raum, der Orientierung seiner optischen Achse 22 und seiner hyperfokalen Distanz D ein vorbestimmtes Soll in Relation zur Position des Arbeitspunktes 26 und der Orientierung der Arbeitsachse 24 des Instruments erfüllt. In diesem Zustand sind folgende Kriterien des Solls erfüllt: der Arbeitspunkt 26 ist im Zentrum des Sichtfeldes 30 angeordnet, der Arbeitspunkt 26 fällt mit der fokalen Distanz D zusammen und ist in einer Bildebene 27 angeordnet. Zudem weist die optische Achse 22 den vorbestimmten Winkel W relativ zur Arbeitsachse 24 auf.

Prinzipiell erfolgt die optische Erfassung des zuvor beschriebenen optischen Musters 32 fortwährend mittels der Erstellung der Aufnahme durch das Visualisierungssystem 18. Auf Basis der Aufnahme ermittelt das offenbarungsgemäße Trackingsystem durch bildverarbeitende Auswertung der Aufnahme und des darin enthaltenen Musters 32 mit einem Algorithmus maschinellen Sehens die Position des Arbeitspunkt des 26 und die Orientierung der Arbeitsachse 24. Die Steuereinheit 28 überprüft fortwährend, ob das Visualisierungssystem 18 das oben genannte Soll erfüllt, was im Grundzustand gemäß Figur 4a der Fall ist.

Figur 4b zeigt einen Zustand, in welchem das Instrument 20 aus der gemäß Figur 4a vorbeschriebenen Position und Orientierung ausgelenkt ist. Dem entsprechend ermittelt das offenbarungsgemäße Trackingsystem die geänderte Position und Orientierung des Arbeitspunktes 26 und der Arbeitsachse 24. In Folge ermittelt die Steuereinheit 28 entsprechende Abweichungen des Sichtfeldes 30, der Orientierung der optischen Achse 22 und hyperfokalen Distanz D vom oben genannten Soll.

In Abhängigkeit der Abweichungen ermittelt die Steuereinheit 28 eine notwendige Translation T und Rotation R, in einem oder in mehreren translatorischen, beziehungsweise rotatorischen Freiheitsgraden, um die Abweichung vom Soll durch Ansteuerung des Roboters auszugleichen.

Gemäß Figur 4c wird über die Steuereinheit 28 der Roboter zunächst so angesteuert, dass das Visualisierungssystem 18 allein translatorisch bewegt wird, sodass zunächst der Arbeitspunkt 26 ins Zentrum des Sichtfeldes 30 gerückt wird und eine Koinzidenz des Arbeitspunktes 26 mit der hyperfokalen Distanz D hergestellt wird.

Im gezeigten Ausführungsbeispiel des Assistenzsystems 1 erfolgt die Ansteuerung des Roboters bezüglich der Translation T und Rotation R sequenziell, alternativ kann sie natürlich synchron erfolgen, sodass die notwendige Translation T und Rotation R gleichzeitig ausgeführt wird.

Nach der sequenziell ausgeführten Translation T gemäß Figur 4c weisen die optische Achse 22 und die Arbeitsachse 24 weiterhin einen vom Soll W abweichenden, kleineren Winkel w auf. Die Winkelabweichung vom Soll W wird in Folge gemäß Figur 4d ausgeglichen, indem die Steuereinheit 28 den Roboter derart ansteuert, dass er die notwendige Rotation R ausführt.

Das Ausgleichen der Abweichung vom Soll mittels der notwendigen Translation T und Rotation R kann automatisiert fortwährend, bzw. kontinuierlich erfolgen, wie es beispielsweise anhand der Figuren 4a bis 4a beschrieben wurde, oder es erfolgt nur punktuell auf Anforderung. Ein hierfür notwendiges Anforderungssignal kann beispielsweise von einem Benutzer ausgelöst werden, in dem dieser ein Betätigungselement, beispielsweise ein Pedal oder einen Knopf betätigt, das in seinem Wirkungsfeld angeordnet ist. Beispielsweise kann das Instrument 20 ein solches Betätigungselement als Bedienschnittstelle aufweisen. Alternativ kann das Anforderungssignal durch eine Geste oder einen Stimmbefehl ausgelöst werden.

Der vorbestimmte Winkel W kann in Abhängigkeit von Präferenzen des Benutzers voreingestellt werden und bedarfsgerecht angepasst oder verändert werden. Der Winkel W verhindert, dass der Schaft des Instrumentes 20 das Sichtfeld 30 des Mikroskops in zu großem Umfang verblockt oder teilerblockt.

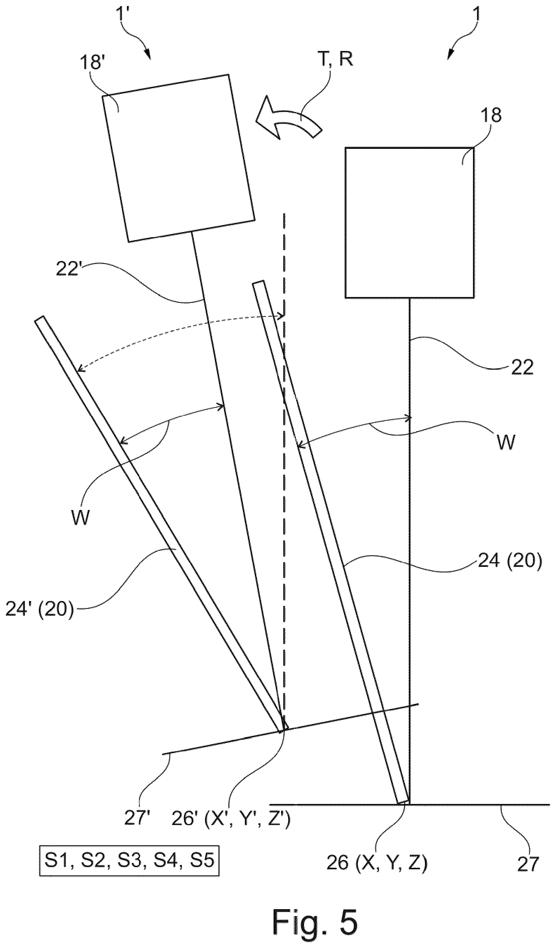

Figur 5 zeigt den Vorgang des oben skizzierten Verfahrens in vergrößerter Darstellung. Der Grundzustand des Assistenzsystems 1 ist in Figur 5 rechts vom Visualisierungssystem 18, der optischen Achse 22, der orthogonal zur optischen Achse 22 angeordneten Bildebene 27, der Arbeitsachse 24 des Effektors 20, des Arbeitspunktes 26 in seinen Koordinaten X, Y, Z und repräsentiert. Dabei ist die optische Achse 22 gemäß dem Soll mit dem vorbestimmten Winkel W gegen die Arbeitsachse 24 abgewinkelt, der Arbeitspunkt 26 fällt mit der hyperfokalen Distanz zusammen und liegt somit im Zentrum des Sichtfeldes und in der Bildebene 27.

Es erfolgt eine Veränderung einer Position und Lage des Instrumentes 20 gemäß Figur 5 in eine neue Position des Arbeitspunktes 26' mit seinen neuen Koordinaten X', Y' und Z', was einer Translation T entspricht, und in eine neue Orientierung der Arbeitsachse 24' des Instrumentes 20', was einer Rotation R entspricht.

Das Visualisierungssystem 18 erstellt kontinuierlich die Aufnahme, in der sich die Transformation des Instrumentes 20 (Translation T und Rotation R) in der optisch erfassten Veränderung oder Verzerrung des Musters 32 zeigt.

Das Trackingsystem ermittelt mittels Bildverarbeitung der Aufnahme, das heißt mittels dem Algorithmus maschinellen Sehens, die neue Lage oder Orientierung des Instrumentes 20 und stellt sie der Steuereinheit 28 bereit.

Die Steuereinheit 28 ermittelt hieraus die zum Erreichen des Solls notwendige Translation T und Rotation R des Visualisierungssystems 18. Sobald der Operateur das Nachsteuern des Visualisierungssystems 18 durch ein geeignetes Anforderungssignal auslöst, steuert die Steuereinheit 28 den Roboter derart an, dass er das Assistenzsystem 1' in die von den Bezugszeichen 1', 18', 22', 24', 27' repräsentierte Konstellation bewegt.

### Bezugszeichenliste

- 1: chirurgisches Assistenzsystem
- 2: Roboter
- 4: Roboterbasis
- 6, 8, 10: Roboterarm-Segment
- 12, 14: Gelenk
- 16: Endabschnitt Roboterarm
- 18: Visualisierungssystem
- 20: Effektor
- 22: Optische Achse
- 24: Arbeitsachse
- 26: Arbeitspunkt
- 27: Bildebene
- 28: Steuereinheit
- 30: Sichtfeld
- 32: optische Referenzmarke

- S1: Schritt Tracking Visualisierungssystem
- S2: Schritt Erstellen Aufnahme
- S3: Schritt Optisches Erfassen Referenzmarke
- S4: Schritt Ermitteln Lage Effektor
- S5, S5.1, S5.21, S5.22: Schritt Steuern Visualisierungssystem

## Patentansprüche

1. Chirurgisches Assistenzsystem (1) mit einem chirurgischen Roboter (2) zur Verwendung für einen chirurgischen Eingriff, mit:
einer Roboterbasis (4) als lokalen Anbindungspunkt des Roboters (2) und einem an die Roboterbasis (4) angebundenen, bewegbaren Roboterarm mit zumindest einem Roboterarm-Segment (6, 8, 10),
einem Visualisierungssystem (18), insbesondere mit einem Operationsmikroskop und/oder Endoskop, das an dem Roboterarm angebunden und dafür angepasst ist, zumindest eine zeitaktuelle Aufnahme zu erstellen und zeitaktuell bereitzustellen,
einem chirurgischen Effektor (20), insbesondere einem chirurgischen Instrument, der insbesondere manuell geführt ist und einen auf oder bei seiner Arbeitsachse (24) angeordneten Arbeitspunkt (26) hat, der bestimmungsgemäß in einem Sichtfeld (30) des Visualisierungssystems (18) angeordnet ist oder anordenbar ist,
einer optischen Referenzmarke, die an dem Effektor (20) in einer mit Bezug zum Arbeitspunkt (26) vorbestimmten Position, insbesondere Position und Orientierung, angeordnet ist, und die dafür angepasst ist, dass auf Basis ihrer optischen Erfassung eine Position des Arbeitspunktes (26) und/oder eine Orientierung der Arbeitsachse (24) ermittelbar ist, wobei die Referenzmarke von einem optischen Muster (32) gebildet ist, das sich entlang zumindest eines Oberflächenabschnitts des Effektors (20) erstreckt,
einem Trackingsystem, das dafür angepasst ist, die Position des Arbeitspunktes (26) und/oder die Orientierung der Arbeitsachse (24) in Abhängigkeit des optisch erfassten Musters (32) zu ermitteln, insbesondere mittels maschinellem Sehen auf Basis der Aufnahme in Bezug zu einem Koordinatensystem des Visualisierungssystems, und
einer Steuereinheit (28), die dafür angepasst ist, eine Position des Visualisierungssystems (18) und/oder eine Orientierung einer optischen Achse (22) des Visualisierungssystems (18) und/oder eine hyperfokale Distanz (D) des Visualisierungssystems (18) in Abhängigkeit der ermittelten Position des Arbeitspunktes (26) und/oder der ermittelten Orientierung der Arbeitsachse (24) zu steuern,
**dadurch gekennzeichnet, dass** die Steuereinheit (28) dafür angepasst ist, die Position des Visualisierungssystems (18) und/oder die Orientierung der optischen Achse (22) des Visualisierungssystems (18) und/oder die hyperfokale Distanz (D) des Visualisierungssystems (18) in Abhängigkeit der ermittelten Position des Arbeitspunktes (26) und/oder der ermittelten Orientierung des Arbeitsachse (24) derart zu steuern, dass bei fester Position des Visualisierungssystems die hyperfokale Distanz (D) auf den Arbeitspunkt (26) eingestellt wird, oder dass bei fester hyperfokaler Distanz (D) die Position des Visualisierungssystems (18) so eingestellt wird, sodass die hyperfokale Distanz (D) mit dem Arbeitspunkt (26) zusammenfällt.

2. Chirurgisches Assistenzsystem (1) nach Anspruch 1, **dadurch gekennzeichnet, dass** die Steuereinheit (28) dafür angepasst ist, in einem Nachverfolgungsmodus das Visualisierungssystem (18) derart zu bewegen, dass die Position des Arbeitspunktes (26) des Effektors (20) mit einem Mittelpunkt oder einem vordefinierten Punkt der Aufnahme zusammenfällt, sodass das Visualisierungssystem (18) dem Arbeitspunkt (26) des Effektors (20) folgt, und dass die optische Achse (22) einen vorbestimmten Winkel (W), insbesondere eine statische Relation, zu der Arbeitsachse (24) aufweist und diesen bei Bewegung des Effektors (20) beibehält.

3. Chirurgisches Assistenzsystem (1) nach einem der vorhergehenden Ansprüche, **dadurch gekennzeichnet, dass** die Steuereinheit (28) dafür angepasst ist, das Visualisierungssystem (18) bezüglich seiner Position und/oder Orientierung seiner optischen Achse (22) und/oder seiner hyperfokalen Distanz (D) fortwährend/kontinuierlich zu steuern oder auf Anforderung/Eingabe, insbesondere in Abhängigkeit wenigstens eines Anforderungssignals, zu steuern.

4. Chirurgisches Assistenzsystem (1) nach einem der vorhergehenden Ansprüche, **dadurch gekennzeichnet, dass** die Steuereinheit (28) dafür angepasst ist, ein durch die (initiale) optische Achse (22) und die neue Arbeitsachse (24'), aufgespanntes, flächiges Band, insbesondere aufgespannte Ebene, zu bestimmen, und in diesem Band die neue optische Achse (22') mit einem vorbestimmten Winkel (W) zu der neuen Arbeitsachse (24') festzulegen.

5. Chirurgisches Assistenzsystem (1) nach einem der vorstehenden Ansprüche, **dadurch gekennzeichnet, dass** der Effektor (20) mit einer festen, nicht wechselbaren Funktionseinheit ausgebildet ist, an der der Arbeitspunkt (26) angeordnet ist, oder wobei der Effektor (20) eine Wechselaufnahme hat, in die einer von mehreren möglichen Funktionseinheiten wahlweise einsetzbar ist, an denen der jeweilige Arbeitspunkt (26) angeordnet ist, wobei die Wechselaufnahme das Muster (32) aufweist.

6. Chirurgisches Assistenzsystem (1) nach Anspruch 5, **dadurch gekennzeichnet, dass** das Muster (32) und/oder Abschnitte des Musters einen jeweils vorbestimmten Abstand zu dem Arbeitspunkt (26) hat oder haben, der insbesondere in einer Speichereinheit hinterlegt ist.

7. Chirurgisches Assistenzsystem (1) nach einem der vorhergehenden Ansprüche, **dadurch gekennzeichnet, dass** das Muster (32) zumindest in Richtung der Arbeitsachse (24) eine Variation aufweist, insbesondere das Muster (32) Elemente (34, 36, 38) hat, die in Richtung der Arbeitsachse (24) beabstandet sind, wobei insbesondere ein Abstand der Elemente (34, 36, 38) untereinander und/oder eine jeweilige Erstreckung der Elemente (34, 36, 38) in Richtung der Arbeitsachse (24) eine Variation aufweist oder aufweisen.

8. Chirurgisches Assistenzsystem (1) nach Anspruch 6 oder 7, **dadurch gekennzeichnet, dass** wenigstens einige der Elemente (34, 36, 38) zu Gruppen (44, 46, 48) zusammengefasst sind, innerhalb derer die Elemente (34, 36, 38) in Bezug auf ihren Abstand untereinander und/oder ihrer jeweiligen Erstreckung in Richtung der Arbeitsachse (24) gleich sind, insbesondere der Abstand der Elemente (34, 36, 38) untereinander und/oder die Erstreckung der Elemente (34, 36, 38) über die Gruppen (44, 46, 48) hinweg eine Variation aufweist oder aufweisen.

9. Chirurgisches Assistenzsystem (1) nach einem der vorhergehenden Ansprüche, **dadurch gekennzeichnet, dass**
sich das Muster (32) vollumfänglich um die Arbeitsachse (24) erstreckt, und/oder
das Muster (32) zu der Arbeitsachse (24) zumindest abschnittsweise rotationssymmetrisch ausgebildet ist.

10. Chirurgisches Assistenzsystem (1) nach einem der vorhergehenden Ansprüche, **dadurch gekennzeichnet, dass** das Visualisierungssystem (18) dafür angepasst ist, die Aufnahme dem Trackingsystem bereitzustellen, und wobei das Trackingsystem dafür angepasst ist, die Position des Arbeitspunktes (26) und/oder die Orientierung der Arbeitsachse (24) des Effektors (20) in Abhängigkeit der Aufnahme zu ermitteln.

## Claims

1. A surgical assistance system (1) comprising a surgical robot (2) for use for a surgical intervention, with:
a robot base (4) as a local link point of the robot (2) and a movable robot arm connected to the robot base (4) with at least one robot-arm segment (6, 8, 10),
a visualization system (18), in particular with a surgical microscope and/or endoscope, which is connected to the robot arm and adapted to create and to real-time provide at least one real-time image,
a surgical effector (20), in particular a surgical instrument, which in particular is manually guided and has a working point (26) arranged on or near its working axis (24) and which is arranged or arrangeable as intended in a field of view (30) of the visualization system (18),
an optical reference marker which is arranged on the effector (20) in a predetermined position with respect to the working point (26), in particular in a position and orientation, and which is adapted to determine a position of the working point (26) and/or an orientation of the working axis (24) on the basis of its optical detection, wherein the reference marker is formed by an optical pattern (32) which extends along at least a surface portion of the effector (20),
a tracking system which is adapted to determine the position of the working point (26) and/or the orientation of the working axis (24) as a function of the optically detected pattern (32), in particular via machine vision on the basis of the image in relation to a coordinate system of the visualization system, and
a control unit (28) which is adapted to control a position of the visualization system (18) and/or an orientation of an optical axis (22) of the visualization system (18) and/or a hyperfocal distance (D) of the visualization system (18) as a function of the determined position of the working point (26) and/or of the determined orientation of the working axis (24),
**characterized in that** the control unit (28) is adapted to control the position of the visualization system (18) and/or the orientation of the optical axis (22) of the visualization system (18) and/or the hyperfocal distance (D) of the visualization system (18) as a function of the determined position of the working point (26) and/or of the determined orientation of the working axis (24) such that, when the position of the visualization system is fixed, the hyperfocal distance (D) is set to the working point (26), or that when the hyperfocal distance (D) is fixed, the position of the visualization system (18) is set such that the hyperfocal distance (D) coincides with the working point (26).

2. The surgical assistance system (1) according to claim 1, **characterized in that** the control unit (28) is adapted to move the visualization system (18) in a tracking mode such that the position of the working point (26) of the effector (20) coincides with a center point or a predefined point of the image, so that the visualization system (18) follows the working point (26) of the effector (20), and that the optical axis (22) has a predetermined angle (W), in particular a static relation, to the working axis (24) and maintains this during movement of the effector (20).

3. The surgical assistance system (1) according to one of the preceding claims, **characterized in that** the control unit (28) is adapted to constantly/continuously control the visualization system (18) with respect to its position and/or orientation of its optical axis (22) and/or its hyperfocal distance (D) or to control it on request/input, in particular as a function of at least a request signal.

4. The surgical assistance system (1) according to one of the preceding claims, **characterized in that** the control unit (28) is adapted to determine a flat band, in particular a spanned plane, spanned by the (initial) optical axis (22) and the new working axis (24'), and to define the new optical axis (22') in this band with a predetermined angle (W) to the new working axis (24').

5. The surgical assistance system (1) according to one of the preceding claims, **characterized in that** the effector (20) is configured with a fixed, non-interchangeable functional unit, on which the working point (26) is arranged, or wherein the effector (20) has an interchangeable holder, into which one of several possible functional units can be selectively inserted, on which the respective working point (26) is arranged, wherein the interchangeable holder comprises the pattern (32).

6. The surgical assistance system (1) according to claim 6, **characterized in that** the pattern (32) and/or portions of the pattern has or have a respectively predetermined distance from the working point (26), which is stored in particular in a memory unit.

7. The surgical assistance system (1) according to one of the preceding claims, **characterized in that** the pattern (32) has a variation at least in the direction of the working axis (24), in particular the pattern (32) has elements (34, 36, 38) that are spaced apart in the direction of the working axis (24), wherein in particular a distance of the elements (34, 36, 38) from each other and/or a respective extension of the elements (34, 36, 38) in the direction of the working axis (24) has or have a variation.

8. The surgical assistance system (1) according to claim 6 or 7, **characterized in that** at least some of the elements (34, 36, 38) are combined to groups (44, 46, 48) within which the elements (34, 36, 38) are the same with respect to their distance from each other and/or their respective extension in the direction of the working axis (24), in particular the distance of the elements (34, 36, 38) from each other and/or the extension of the elements (34, 36, 38) across the groups (44, 46, 48) has or have a variation.

9. The surgical assistance system (1) according to one of the preceding claims, **characterized in that**
the pattern (32) extends completely around the working axis (24), and/or
the pattern (32) is at least partially rotationally symmetrical to the working axis (24).

10. The surgical assistance system (1) according to one of the preceding claims, **characterized in that** the visualization system (18) is adapted to provide the image to the tracking system, and wherein the tracking system is adapted to determine the position of the working point (26) and/or the orientation of the working axis (24) of the effector (20) as a function of the image.

## Revendications

1. Système d'assistance chirurgicale (1) avec un robot chirurgical (2) destiné à l'utilisation pour une intervention chirurgicale avec :
une base de robot (4) comme point de liaison local du robot (2) et un bras de robot mobile lié à la base de robot (4) avec au moins un segment de bras de robot (6, 8, 10),
un système de visualisation (18), en particulier avec un microscope d'opération et/ou un endoscope qui est lié au bras de robot et adapté afin d'établir et de fournir en temps réel au moins une image en temps réel,
un effecteur chirurgical (20), en particulier un instrument chirurgical qui est guidé en particulier manuellement et présente un point de travail (26) agencé sur ou près de son axe de travail (24) qui est ou peut être agencé selon les dispositions dans un champ de vision (30) du système de visualisation (18),
une marque de référence optique qui est agencée au niveau de l'effecteur (20) dans une position prédéterminée par rapport au point de travail (26), en particulier une position et orientation et qui est adaptée afin que sur la base de leur détection optique, une position du point de travail (26) et/ou une orientation de l'axe de travail (24) puisse être déterminée, dans lequel la marque de référence est formée par un modèle optique (32) qui s'étend le long d'au moins une section de surface de l'effecteur (20),
un système de suivi qui est adapté afin de déterminer la position du point de travail (26) et/ou l'orientation de l'axe de travail (24) en fonction du modèle (32) détecté optiquement, en particulier au moyen d'une vision artificielle sur la base de l'image par rapport à un système de coordonnées du système de visualisation et
un dispositif de commande (28) qui est adapté afin de commander une position du système de visualisation (18) et/ou une orientation d'un axe optique (22) du système de visualisation (18) et/ou une distance (D) hyperfocale du système de visualisation (18) en fonction de la position déterminée du point de travail (26) et/ou de l'orientation déterminée de l'axe de travail (24),
**caractérisé en ce que** le dispositif de commande (28) est adapté afin de commander la position du système de visualisation (18) et/ou l'orientation de l'axe optique (22) du système de visualisation (18) et/ou la distance (D) hyperfocale du système de visualisation (18) en fonction de la position déterminée du point de travail (26) et/ou de l'orientation déterminée de l'axe de travail (24) de telle manière qu'en cas de position fixe du système de visualisation, la distance (D) hyperfocale soit réglée sur le point de travail (26), ou qu'en cas de distance (D) hyperfocale fixe, la position du système de visualisation (18) soit réglée de sorte que la distance (D) hyperfocale coïncide avec le point de travail (26).

2. Système d'assistance chirurgicale (1) selon la revendication 1, **caractérisé en ce que** le dispositif de commande (28) est adapté afin de déplacer dans un mode de poursuite le système de visualisation (18) de telle manière que la position du point de travail (26) de l'effecteur (20) coïncide avec un point médian ou un point prédéfini de l'image de sorte que le système de visualisation (18) suive le point de travail (26) de l'effecteur (20) et **en ce que** l'axe optique (22) présente un angle (W) prédéterminé, en particulier une relation statique, à l'axe de travail (24) et conserve celui-ci lors du mouvement de l'effecteur (20).

3. Système d'assistance chirurgicale (1) selon l'une quelconque des revendications précédentes, **caractérisé en ce que** le dispositif de commande (28) est adapté afin de commander en permanence/en continu le système de visualisation (18) par rapport à sa position et/ou son orientation de son axe optique (22) et/ou sa distance (D) hyperfocale ou sur demande/saisie en particulier en fonction d'au moins un signal de demande.

4. Système d'assistance chirurgicale (1) selon l'une quelconque des revendications précédentes, **caractérisé en ce que** le dispositif de commande (28) est adapté afin de déterminer une bande plane serrée par l'axe optique (22) initial et le nouvel axe de travail (24') en particulier plan serré et de fixer dans cette bande le nouvel axe (22') optique avec un angle prédéterminé (W) au nouvel axe de travail (24').

5. Système d'assistance chirurgicale (1) selon l'une quelconque des revendications précédentes, **caractérisé en ce que** l'effecteur (20) est formé avec une unité fonctionnelle fixe non interchangeable, au niveau de laquelle le point de travail (6) est agencé, ou dans lequel l'effecteur (20) présente une image interchangeable, dans laquelle une des plusieurs unités fonctionnelles possibles peut être insérée au choix, au niveau desquelles le point de travail (26) respectif est agencé, dans lequel l'image interchangeable présente le modèle.

6. Système d'assistance chirurgicale (1) selon la revendication 5, **caractérisé en ce que** le modèle (32) et/ou des sections du modèle présente ou présentent une distance prédéterminée respectivement par rapport au point de travail (26) qui est enregistrée en particulier dans une unité de mémoire.

7. Système d'assistance chirurgicale (1) selon l'une quelconque des revendications précédentes, **caractérisé en ce que** le modèle (32) présente au moins en direction de l'axe de travail (24) une variation, en particulier le modèle (32) présente des éléments (34, 36, 38) qui sont espacés en direction de l'axe de travail (24), dans lequel en particulier une distance des éléments (34, 36, 38) entre eux et/ou une étendue respective des éléments (34 36, 38) présente ou présentent en direction de l'axe de travail (24) une variation.

8. Système d'assistance chirurgicale (1) selon la revendication 6 ou 7, **caractérisé en ce qu'**au moins certains des éléments (34, 36, 38) sont réunis en groupes (44, 46, 48), à l'intérieur desquels les éléments (34, 36, 38) sont égaux en ce qui concerne leur distance entre eux et/ou leur étendue respective en direction de l'axe de travail (24), en particulier la distance des éléments (34, 36, 38) entre eux et/ou l'étendue des éléments (34, 36, 38) présente ou présentent une variation au-delà des groupes (44, 46, 48).

9. Système de navigation chirurgicale (1) selon l'une quelconque des revendications précédentes, **caractérisé en ce que**
le modèle (32) s'étend sur toute la superficie autour de l'axe de travail (24), et/ou
le modèle (32) est formé à symétrie de rotation au moins par section par rapport à l'axe de travail (24).

10. Système d'assistance chirurgicale (1) selon l'une quelconque des revendications précédentes, **caractérisé en ce que** le système de visualisation (18) est adapté afin de fournir l'image au système de suivi et dans lequel le système de suivi est adapté afin de déterminer la position du point de travail (26) et/ou l'orientation de l'axe de travail (24) de l'effecteur (20) en fonction de l'image.
